# EUROPEAN PATENT APPLICATION

(11) **EP 1 832 655 A2**
(43) Date of publication of application: **12.09.2007**
(21) Application number: 07008899.2
(22) Date of filing: 29.04.1998
(51) Int. Cl.: C12N 15/12, C07K 14/435, C07K 16/18, C12Q 1/68, G01N 33/53, A61K 31/365, A01N 43/90, C12N 5/10

(54) **Methods for detecting and reversing resistance to macrocyclic lactone compounds**

(30) Priority: 30.04.1997 US 45160 P; 28.04.1998 US 67676
(62) Divisional of application: 98917525.2
(71) Applicant: McGill University, Montreal, QC H3A 2T5 (CA)
(72) Inventor: Prichard, Roger K., Beaconsfield Quebec H9W 4K5 (CA); Xu, Ming, Chino CA 91710 (US); Ribeiro, Ana Paula, Montreal Quebec H4A 2W2 (CA); Blackhall, William J., Pointe Claire Quebec H9R 3H4 (CA); Beech, Robin N., Beaconsfield Quebec H9W 5A7 (CA); Molento, Marcelo, Sainte-Anne-de-Bellevue Quebec H9X 3V9 (CA); Liu, Hao Yuan, Sainte-Anne-de-Bellevue Quebec H9X 3V9 (CA)
(74) Representative: Blodig, Wolfgang

(57) **Abstract**

A method for increasing the efficacy of a macrocyclic lactone compound against a resistant crop pest characterized by applying to the crop, to the crop seed or to the soil or water in which the crop or the seed is growing or is to be grown a pesticidal enhancing effective amount of a multidrug resistance reversing agent.

## Description

### BACKGROUND OF THE INVENTION

### Related U.S. Application Data

This application claims the benefit under 35 U.S.C. § 119(e) of U.S. Provisional Application No. 60/045,160, filed April 30, 1997.

### Field of the Invention

This invention relates generally to novel methods for diagnosing and overcoming resistance to the macrocyclic lactone compounds. More specifically, the invention pertains to unique methods for detecting the development of resistance to macrocyclic lactones using nucleic acid probes and enhancing the efficacy of the macrocyclic lactones using multidrug resistant reversing agents.

### Description of Related Art

Macrocyclic lactone compounds such as the LL-F28249 compounds, the milbemycins and the avermectins are widely used for treatment of nematode and arthropod parasites. The highly active LL-F28249 family of compounds are natural endectocidal agents isolated from the fermentation broth of *Streptomyces cyaneogriseus* subsp. *noncyanogenus.* U.S. Patent No. 5,106,994 and its continuation U.S. Patent No. 5,169,956 describe the preparation of the major and minor components, LL-F28249α-λ. The LL-F28249 family of compounds further includes, but is not limited to, the semisynthetic 23-oxo derivatives and 23-imino derivatives of LL-F28249α-λ which are shown in U.S. Patent No. 4,916,154. Moxidectin, chemically known as 23-(O-methyloxime)-LL-F28249α, is a particularly potent 23-imino derivative. Other examples of LL-F28249 derivatives include, but are not limited to, 23-(O-methyloxime)-5-(phenoxyacetoxy)-LL-F28249α,23-(semicarbazone)-LL-F28249α and 23-(thiosemicarbazone)-LL-F28249α.

The milbemycins, also known as the B-41 series of antibiotics, are naturally occurring macrocyclic lactones isolated from the microorganism, *Streptomyces hygroscopicus* subsp. *aureolacrimosus.* U.S. Patent No. 3,950,360 shows the preparation of the macrolide antibiotics milbemycin_{α1-α10}, milbemycin_{β1-β3} etc. These compounds are also commonly referred to as milbemycin A, milbemycin B, milbemycin D and the like, or antibiotic B-41A1, antibiotic B-41A3, etc.

The avermectins, also known as the C-076 family of compounds, are naturally occurring macrocyclic lactones produced by the soil actinomycete microorganism, *Streptomyces avermitilis.* U.S. Patent No. 4,310,519 discloses the isolation and preparation of the major components A₁ₐ (e.g., avermectin A₁ₐ), A₂ₐ, B₁ₐ and B₂ₐ, and the minor components A_{1b} (e.g., avermectin A_{1b}), A_{2b}, B_{1b} and B_{2b}. The C-076 family additionally embraces the semisynthetic derivatives such as the 22,23-dihydroavermectins described in U.S. Patent No. 4,199,569. The semisynthetic derivatives include, but are not limited to, ivermectin, abamectin, doramectin, eprinomectin and the like.

Resistance to all of the broad spectrum macrocyclic lactone compounds has been encountered in most regions of the world where the compounds are used routinely in animal production. For instance, drug resistance to ivermectin (IVM), chemically known as 22,23-dihydroavermectin B₁ or 22,23-dihydro C-076 B₁ and a commonly used member of the avermectin drug family, has become a widespread problem, particularly in nematodes of sheep, goats and cattle (Shoop, Parasitol. Today 9: 154-159, 1993). In some parts of the world, the survival of commercial animal production is threatened by the development of anthelmintic resistance. Additionally, there is conflicting evidence as to whether ivermectin (avermectin) resistance confers resistance to the related milbemycins or other macrolides (Arena et al., J. Parasitol. 81: 286-294, 1995; Oosthuizen and Erasmus, J. So. African Vet. Assoc. 64: 9-12, 1993; Pomroy and Whelan, Vet. Rec. 132: 416, 1993; Shoop, 1993; Condora et al., Vet. Rec. 132: 651-652, 1993; Pomroy et al., N.Z. Vet. J. 40: 76, 1992; Pankavich et al., Vet. Rec. 130: 241-242, 1992; Craig et al., Vet. Parasitol. 41: 329-333, 1992). The mechanisms of resistance to the avermectins, the milbemycins and other macrocyclic lactone compounds remain unknown.

P-glycoproteins (Pgp) were identified some years ago as proteins involved in multidrug resistance (MDR) of mammalian tumor cells (Julino and Ling, 1976; Gros and Buschman, 1993; Gotteesman and Pastan, 1993). MDR proteins may also be involved in drug resistance in the protozoal parasites *Entamoeba histolytica* (Whirth, Archivos De Investigacion Medica 21(Supp. 1): 183-189, 1990; Samuelson et al., Mol. Biochem. Parasitol. 38: 281-290, 1990), *Leishmania enriietti* (Chow, Mol. Biochem. Parasitol. 60: 195-208, 1993), *L. dononani* (Callahan et al., Mol. Biochem. Parasitol. 68: 145-149, 1994); and *Plasmodium falciparum* (Volkman et al., Mol. Biochem. Parasitol. 57: 203-211, 1993; Cowman et al., J. Cell Biol. 113: 1033-1042, 1991). While many researchers believe that the proposed mechanism for Pgp involvement in drug resistance is that Pgp behaves as a pump to increase drug efflux, Callahan *et al.* (1994) suggested that Pgp may work by decreasing drug influx. However, the whole picture of how Pgp can be responsible for drug resistance is still unclear.

Only recently have Pgp homologs been investigated in nematodes (Sangster, Parasitol. Today 10: 319-322, 1994; Lincke et al., EMBO J. 12: 1615-1620, 1993; Lincke et al., J. Mol. Biol. 228: 701-711, 1992). Three full length Pgp genes and one partial Pgp gene from the free-living nematode, *Caenorhabditis elegans* have been cloned, sequenced and mapped to chromosomes I, IV and X (Lincke *et al.,* 1992). Sangster et al., J. Cell Biochem. 17 (Supp.): 1223, 1993, indicated evidence for several partial genes for Pgp in the parasitic nematode *Haemonchus contortus,* although sequence information was missing. *In vivo* experiments have shown that disruption of the mouse *mdr*1*,* a P-glycoprotein gene, leads to an impairment in the blood-brain barrier and to increased sensitivity to drugs in these mice (Schinkel et al., Cell 77:491-502, 1994). Mice with deletion of *mdr*1a were 50-100 times more sensitive to ivermectin than normal mice.

Drug resistance based on overexpression of P-glycoprotein has been shown to be reversed by verapamil and a number of other calcium channel blockers, calmodulin antagonists, steroids and hormonal analogs, cyclosporins, dipyridamole and other MDR-reversing agents (Ford, Hematol. Oncol. Clin. North Am. 9: 337-361, 1995). However, there has been no report or suggestion in the literature to use MDR-reversing agents to combat resistance in nematodes and arthropods to pesticides.

There is a definite need to understand the mechanism of macrocyclic lactone resistance, to be able to detect insipient resistance before it becomes flagrant and is difficult to manage the health of the animals. The ability to reverse the resistance has great potential for maintaining parasite control in the face of a failure of conventional treatment. An important object of the present invention, thus, is to determine these mechanisms of resistance in order to find viable, sensitive means to detect and to overcome the problematic resistance thereby improving parasite control.

### BRIEF SUMMARY OF THE INVENTION

Heretofore unknown, it is now found that the mechanism of resistance to the macrocyclic lactone compounds is due to overexpression of novel P-glycoprotein homologs. It is further newly found that the nucleic acid molecules encoding the P-glycoprotein homologs or the fragments thereof regulating this resistance are useful as unique probes in methods for diagnosing the resistance to the macrocyclic lactones. For the first time, the reversal of resistance to the macrocyclic lactone compounds using multidrug resistance reversing agents is described herein.

### BRIEF DESCRIPTION OF THE DRAWINGS

The background of the invention and its departure from the art will be further described hereinbelow with reference to the accompanying drawings, wherein:
Figure 1 shows the 432 bp PCR product which is generated from a *Haemonchus contortus* cDNA pBLUESCRIPT® library as template and degenerate primers based on the conserved ATP binding domains of *Caenorhabditis elegans* P-glycoprotein genes after electrophoresis on an agarose gel.
Figures 2A and 2B represent, respectively, the nucleotide sequence of the 432 bp PCR product shown in Figure 1 and the predicted amino acid translation of the cDNA (which correspond to SEQ ID NO:1 and SEQ ID NO:2, respectively).
Figure 3 shows the autoradiographs of the Northern blots of RNA extracted from eggs of ivermectin sensitive and resistant (MKIS and MKIR; ACIS and ACIR) nematode strains respectively. The [³²P]-432 bp PCR product, with homology to Pgp, is used as one probe and a [³²P]-actin fragment from *pBA*1 is used as a second probe.
Figures 4A to 4B represent the full-length cDNA sequence (4175 bp) of the PGP-A clone from the *H*. *contortus* cDNA library with high homology to known P-glycoproteins (which corresponds to SEQ ID NO:3).
Figure 5 represents the partial cDNA sequence (1810 bp) of the 5' end of the PGP-A clone from the *H. contortus* cDNA library (which corresponds to SEQ ID NO:4). 1810ut
Figure 6 represents the partial cDNA sequence (2698 bp) of the 3' end of the PGP-A clone from the *H. contortus* cDNA library (which corresponds to SEQ ID NO:5).
Figure 7 represents the putative amino acid translation (1275 a.a.) of PGP-A cDNA (which corresponds to SEQ ID NO:6).
Figures 8A to 8B represent the partial cDNA sequence (3512 bp) of the 3' end of the related but different PGP-O clone from the *H*. *contortus* cDNA library (which corresponds to SEQ ID NO:7).
Figure 9 represents the partial cDNA sequence (2681 bp) of 3' end of the related but different PGP-B clone from the *H*. *contortus* cDNA library (which corresponds to SEQ ID NO:8).
Figure 10 shows the autoradiographs of the Southern blots of genomic DNA extracted from eggs of ivermectin sensitive and resistant strains of *H. contortus* (MKIS AND MKIR) after digestion with *Pvu*II, electrophoresis and probed with the [³²P]-432 bp *H. contortus* Pgp probe.
Figure 11 shows the restriction length polymorphism of PCR products from the DNA of individual male adult worms from ivermectin susceptible (lanes 1-9) or resistant (lanes 11-20) *H. contortus* strains, generated with P-glycoprotein primers PGP2S and PGPAS followed by digestion with *Dde*I and separation on non-denaturing polyacrylamide gel electrophoresis. The arrows point to the three digestion fragments that are associated with resistance.
Figures 12A and 12B represent the nucleic acid sequences comprising sense primer PGP2S (Fig. 12A, which corresponds to SEQ ID NO:9) and antisense primer PGPAS (Fig. 12B, which corresponds to SEQ ID NO:10) which are constructed from the nematode P-glycoprotein homolog cDNA clone PGP-O-3' (53 bp intron region) and are used to generate PCR products that are diagnostic for macrocyclic lactone endectocide resistance.
Figures 13A and 13B illustrate the efficacy of moxidectin (MOX) against *H. contortus* susceptible (Fig. 13A) or moxidectin-resistant (Fig. 13B) strains in jirds.
Figures 14A and 14B illustrate the efficacy of ivermectin (IVM) against *H. contortus* susceptible (Fig. 14A) and moxidectin-resistant (Fig. 14B) strains in jirds.
Figures 15A and 15B illustrate the efficacy of verapamil (VRP) with or without ivermectin (IVM; LD₅₀) against *H*. *contortus* susceptible (Fig. 15A) or moxidectin-resistant (Fig. 15B) strains in jirds.
Figures 16A and 16B illustrate the efficacy of the combination of moxidectin (MOX; Fig. 16A) or ivermectin (IVM; Fig. 16B) with verapamil (VRP) against *H*. *contortus* moxidectin-resistant strain in jirds.
Figures 17A, 17B and 17C illustrate, respectively, the *Hin*fI digestion of P-glycoprotein PCR fragments from the DNA of individual worms of susceptible, ivermectin-resistant and moxidectin-resistant *H. contortus,* using primers PGP2S and PGPAS, followed by digestion and separation on non-denaturing polyacrylamide gel electrophoresis. The arrows on the right side of Figures 17B and 17C point to the digestion fragments that are associated with resistance while the arrows on the left side point to the position and size of the standard markers.
Figures 18A, 18B and 18C illustrate, respectively, the *Alu*I digestion of P-glycoprotein PCR fragments from the DNA of individual worms of susceptible, ivermectin-resistant and moxidectin-resistant *H. contortus,* using primers PGP2S and PGPAS, followed by digestion and separation on non-denaturing polyacrylamide gel electrophoresis. The arrows on the right side of Figures 18B and 18C point to the digestion fragments that are associated with resistance while the arrows on the left side point to the position and size of the standard markers.

### DETAILED DESCRIPTION OF THE INVENTION

In accordance with the present invention, there are provided novel purified and isolated nucleic acid molecules encoding new P-glycoprotein homologs or the fragments thereof which regulate the macrocyclic lactone resistance. These nucleic acids find use as probes in innovative methods for the early diagnosis of a developing resistance to the endectocides. In the past, there have been no DNA or RNA based methods of detection of macrocyclic lactone resistance available. Now, the present invention uniquely provides the genetic basis of the resistance and the diagnosis of resistance using nucleic acid probes. The early detection under the guidance of this invention allows for maintaining adequate control of parasites and maintaining the usefulness of the macrocyclic lactone compounds. Additionally, the mechanism of resistance to macrocyclic lactones can be used in development of screens for identifying new antiparasitic agents.

The novel methods of the present invention which are useful for detecting the resistance to macrocyclic lactone compounds in nematodes or arthropod pests utilize the new nucleic acid probes described herein. A variety of techniques well-known to those versed in the art can be employed for the analysis. Desirably, the method detects changes in genomic DNA or mRNA to provide a viable means for diagnosis of macrocyclic lactone resistance.

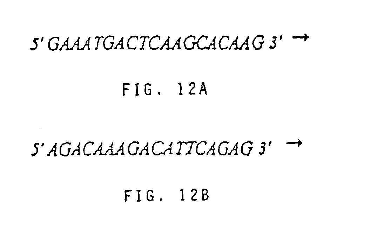

These methods include, for example, Polymerase Chain Reaction (PCR), hybridization in a Southern blot, Dot blot or Northern blot analysis or the use of an antibody to a sequence of peptides corresponding to the translation of the nucleotide sequences between the novel primers of the invention of an individual pest or mixture of the pests such as worms, using primers or probes, for example, corresponding to the portion of the cDNA sequence of PGP-O between the sequences identified as PGP2S and PGPAS (see Figs. 12A and 12B). Alternative primers or probes within this region which can be utilized in the methods of the invention include, but are not limited to, all combinations of PCR primers or probes within this region or that of other PGP homolog sequences such as PGP-A, PGP-B, PGP-O and the like. Basically, the coding region of the P-glycoprotein homolog genes corresponding to the cDNA sequences identified as PGP-A, PGP-A-3', PGP-B, PGP-B-3', PGP-O, PGP-O-3' and the like is detected by PCR, Southern blot, Dot blot, Northern blot, Restriction Fragment Length Polymorphism (RFLP) and other standard means of analysis. Surprisingly, it has been found that the digestion pattern from the PCR fragment, the blot data or the antibody-antigen reaction are associated with susceptible or resistant traits which are diagnostic for the development of macrocyclic lactone resistance.

Polymerase Chain Reaction (PCR) can be employed for the detection of resistance to the macrocyclic lactone compounds by synthesizing a nucleic acid product which can be probed in conjunction with the Southern blot analysis or initially digested with a restriction enzyme for RFLP analysis as described herein. The primers are used to initiate a PCR reaction using the nucleic acids extracted from the pest specimen. They are used to synthesize a P-glycoprotein sequence or sequences. The PCR products can then be cut with restriction enzymes and the digested sequences run on an electrophoresis gel. Examples of suitable restriction enzymes that can be employed in the digestion of the PCR products include, but are not limited to, *Alu*I, *Dde*I*, Hinf*I, *Rsa*I and the like. The pattern of bands observed on a Southern blot or a Northern blot indicates which P-glycoprotein alleles are present in a pest specimen such as the worm or group of worms. Some of the alleles can be associated with macrocyclic lactone sensitivity and others with resistance to macrocyclic lactones. The PCR products, followed by restriction enzyme digests, provide viable means for the detection of resistance. The process of cutting the PCR products or the nucleic acids such as DNA for the RFLP analysis greatly increases the sensitivity and specificity of the diagnosis.

Reverse Transcriptase - Polymerase Chain Reaction analysis (RT-PCR) can similarly be employed for the detection of resistance to the macrocyclic lactone compounds in nematode or arthropod pests. Typically, RNA from a nematode or arthropod specimen is extracted and reverse transcriptase followed by PCR, as described herein, is used to detect resistance.

By way of illustration, the nucleic acids, typically DNA for the PCR procedure or mRNA for RT-PCR, are extracted from the pest specimen, a pest known to be resistant to the macrocyclic lactone compounds and a pest known to be susceptible to the macrocyclic lactones. The nucleic acids derived from the resistant and the susceptible pests are used as a point of reference. The DNA, or cDNA produced by mRNA by Reverse Transcriptase, is denatured and the primers of the invention are added to form a mixture. The three mixtures are subjected to many cycles of PCR, usually digested by a restriction enzyme and subjected to gel electrophoresis. Subsequently, the pattern and the intensity of the bands from the specimen to that of the reference nucleic acids, i.e., DNA or cDNA, of the resistant and susceptible extracts are compared to detect the resistant population. Optionally, hybridization by a probe of the invention or use of a dye such as ethidium bromide to assist in visualizing the bands is included in the process.

Novel probes are used in the diagnosis of macrocyclic lactone resistance by detecting susceptibility or resistance to the macrocyclic lactones in the PCR assay. The primers which are used in the PCR assay are constructed, for example, from the nucleic acid sequences for the parasite P-glycoprotein homolog cDNA clones. Examples of suitable PCR primers that can be employed in the PCR analysis are the primers PGP2S and PGPAS used in the sense and antisense directions, respectively, which are constructed from PGP-O-3' or PGP-O (see Figs. 12A and 12B). The primers can also be prepared from the full or partial sequences of other P-glycoprotein nucleic acids such as PGP-A, PGP-A-3', PGP-B-3', PGP-O, etc. and the complementary strands thereof which contain the region found to be diagnostic of macrocyclic lactone resistance. Alternative useful sequences can be obtained by conventional means such as hybridization techniques under standard or stringent conditions.

Southern blot, Dot blot or Northern blot may be prepared with the nucleic acid molecules from the nematode or the arthropod specimen and, using a probe comprising one of the nucleic acid molecule sequences encoding for resistance or portion thereof, one can compare the level of the nucleic acids extracted from the specimen to the level of the nucleic acids from the probe, for example, by measuring or detecting the level of DNA or mRNA. Generally, three nucleic acid extracts are mapped to make the comparison: from the pest specimen, from a pest known to be resistant and from a pest known to be susceptible. In the case of the Southern blot, the pattern of the bands is compared. With the Northern blot, either the pattern or the intensity of the bands is compared. For the Dot blot, the intensity of the spots is compared.

Another technique involves conducting a Restriction Fragment Length Polymorphism analysis (RFLP) by extracting the nucleic acids from a nematode or arthropod specimen, digesting the nucleic acid with a restriction enzyme, using a probe comprising one of the nucleic acid molecule sequences encoding for resistance or portion thereof and comparing the digestion pattern to that of the digestion pattern of nematodes or arthropods known to be from populations either resistant or sensitive to the macrocyclic lactone endectocides. When DNA is cut with the restriction enzyme, run on a gel and probed under the RFLP technique, the probe hybridizes with the similar sequences, but their length will vary depending upon where the restriction sites for that enzyme occurs. By repeating the analysis with DNA from individual worms, slightly different patterns are observed due to polymorphism. Specific patterns are diagnostic for the resistance gene. *PvuII* is an example of a preferred restriction enzyme that can be employed for RFLP analysis. Other conventional restriction enzymes known to those of ordinary skill in the art may be substituted in the method.

A further example of a process useful in the present invention for detecting resistance concerns making antibodies which employ the novel PGP protein homologs. For instance, an antibody may be prepared to a sequence of the peptide corresponding to the amino acid translation of the nucleic acids or the fragment thereof encoding the P-glycoprotein homologs which regulate resistance. Then, a specimen of the nematode or the arthropod pest, or the extract thereof, is prepared for reaction with the above antibody. The specimen or the extract is reacted with the antibody under suitable conditions that allow antibody-antigen binding to occur and, thereafter, the presence of the antibody-antigen binding is detected by conventional methods.

The above-described methods for the detection of resistance to the macrocyclic lactone compounds can optionally use a P-glycoprotein specific ligand or dye. Usually, the level of the P-glycoprotein in the specimen can more easily be observed using the ligand or dye and compared to the levels obtained in known macrocyclic lacrone resistant and susceptible populations of nematodes or arthropods. The ligand or dye is usually radiolabelled so that it can be readily detected. Examples of suitable ligands useful in this method include, but are not limited to, prazosin, azidoprazosin, iodoaryl-azidoprazosin and the like. A variety of conventional dyes may be employed such as, for instance, rhodamine 123, ethidium bromide and others.

For purposes of this invention, the nucleic acid molecule may be DNA, cDNA or RNA. However, in the most preferred embodiment of this invention, the nucleic acid probe is a cDNA molecule. Many of the foregoing methods illustrate extracted nucleic acids from *Haemonchus contortus.* It is contemplated that the present invention embraces the use of recombinant nucleic acids encoding for resistance or susceptibility to the macrolides as well as isolated nucleic acids from other worm strains or pest species.

The plasmids containing cDNA derived from *Haemonchus contortus* are deposited in connection with the present patent application and maintained pursuant to the Budapest Treaty in the American Type Culture Collection (ATCC), 12301 Parklawn Drive, Rockville, Maryland 20852, U.S.A. The cDNA sequences described herein are contained within plasmids (pBLUESCRIPT® II, commercially available from Stratagene Inc., La Jolla, CA) transformed into XLI-blue *Escherichia coli* bacterial strains. The plasmids identified as PGP-B-3', PGP-O-3' and PGP-A-5' have been deposited in the ATCC on January 29, 1997 and have been assigned ATCC Designation Numbers 98307, 98309 and 98310, respectively. The plasmid PGP-A-3' has been deposited in the ATCC on February 26, 1997 and has been assigned ATCC Designation Number 98336. It should be appreciated that other plasmids, which may be readily constructed using site-directed mutagenesis and the techniques described herein, are also encompassed within the scope of the present invention.

The present invention further relates to the unique reversal of resistance in parasites to the macrocyclic lactone compounds by administering or applying multidrug resistance reversing agents. This reversal of an existing resistance problem permits regaining satisfactory parasite control. The nematode or arthropod parasites or pests of this invention refer to crop insects, crop or mammalian nematodes, arthropod ectoparasites and endoparasites of mammals including acarids and the like.

Desirably, the multidrug resistance reversing agent is a calcium channel blocker such as verapamil, nifedipine and the like; a calmodulin antagonist such as trifluoperazine, prochlorperazine and the like; a vinca alkaloid analog such as vindoline, thaliblastine and the like; a steroidal agent such as progesterone and the like; a hormonal agent such as tamoxifen, estradiol and the like; an immunosuppressive agent such as cyclosporin A, SDZ-PSC 833 and the like, an antibiotic such as erythromycin, cefoperazone, ceftriaxone, tetracycline and the like; miscellaneous compounds such as dipyridamole, quinidine, reserpine, amiodarone, etc.; and other multidrug resistance reversing agents known to those versed in the art.

To increase the efficacy of the parasiticidal macrolides, the compounds of the invention are administered to mammals orally, parenterally, topically (local activity) or transdermally (systemic activity) depending upon the bioavailability of the selected medicinal by the desired route of administration. Parenteral administration of the medicinals encompasses any means other than orally, such as, for example, intravenously, intramuscularly, subcutaneously, intratracheally, intraruminally, etc. It is apparent that the MDR-reversing agents are administered in connection with the administration of the macrocyclic lactone compound encountering resistance in the nematodes or the arthropod ectoparasites or endoparasites of mammals. However, the administration of the MDR-reversing agents may be made either before or during concurrent administration of the macrocyclic lactones. If the MDR-reversing agent will be given before the endectocide, medical or veterinary personnel can readily determine by appropriate blood levels how far in advance the MDR-reversing agent may be given for increasing the macrolide's efficacy. Typically, the MDR-reversing agent will be administered within 24 hours of the start of endectocidal therapy and, preferably, within 4 hours before or concomitantly with administering the macrocyclic lactone.

In terms of dosage, the suitable amount of the MDR-reversing agent which is effective to increase the efficacy of the macrocyclic lactone compound against resistant nematodes or resistant arthropod ectoparasites or endoparasites will typically vary within a wide range of amounts at a variety of concentrations. The particular MDR-reversing agent selected for use with the specific endectocide will clearly affect the useful dose of the MDR-reversing agent. It is contemplated that selection of appropriate dosages of each MDR-reversing agent and the macrocyclic lactone compound to achieve the pesticidal enhancing effective amount can be easily titrated by routine testing known to those having ordinary skill in the medical and veterinary arts.

For use in parasiticidal treatment, the macrocyclic lactone compounds may be administered orally in a unit dosage form such as a capsule, a bolus or a tablet. The capsules and boluses comprise the active ingredient admixed with a conventional carrier vehicle such as starch, talc, magnesium stearate or dicalcium phosphate. The dry, solid unit dosage form are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be widely varied with respect to their total weight and content of the active agent depending upon factors such as the type and the weight of the mammal to be treated and the type and severity of the infection or infestation. Generally, the amount of the macrocyclic compound given in oral administration is about 0.001 mg to about 10 mg per kg of body weight and preferably, about 1 mg to about 5 mg per kg of body weight. However, the amount will vary depending upon the extent of the resistance already developed in the parasite.

For animals, the macrocyclic lactone compound and many of the MDR-reversing agents can also be administered via an animal feedstuff by intimately dispersing the active ingredient in the feed or using as a top dressing or in the form of pellets which may then be added to the finished feed or optionally fed separately. Suitable compositions include feed premixes or supplements in which the active compound is present in relatively large amounts, wherein said feed premixes or supplements are suitable for direct feeding to the animal or for addition to the feed either directly or after an intermediate dilution or blending step.

Typical carriers or diluents suitable for such compositions include distillers' dried grains, corn meal, citrus meal, fermentation residues, ground oyster shells, wheat products, molasses, corn cob meal, edible bean mill feed, soya grits, crushed limestone and the like. The active compounds are intimately dispersed throughout the carrier by methods such as grinding, stirring, milling or tumbling. Compositions containing about 0.005% to about 2.0%, by weight, of the active compound are particularly suitable as feed premixes.

Feed supplements, which are fed directly to the animal, contain about 0.0002% to 0.3%, by weight, of the active compounds. Such supplements are added to the animal feed in an amount to give the finished feed the concentration of active compound desired for the treatment or control of the resistant parasitic disease. Although the desired concentration of the active compound will vary depending upon a variety of factors such as the particular compound employed or the severity of the affliction, the macrocyclic compounds of this invention are usually fed at concentrations of about 0.00001% to about 0.02% in the feed.

Alternatively, the compounds of the present invention may be administered to the afflicted mammals parenterally, in which event the active ingredient is dissolved, dispersed or suspended in a sterile, isotonic, nontoxic liquid carrier vehicle. The active material is admixed with the nontoxic pharmaceutically acceptable vehicle, preferably a vegetable oil such as peanut oil, cotton seed oil or the like. Other parenteral vehicles such as propylene glycol, glycerol and the like may also be used for parenteral formulations.

In the parenteral formulations, the active macrolides are typically dissolved or suspended in the formulation in sufficient amount to provide from about 0.005% to about 5.0%, by weight, of the active compound in said formulation.

Conveniently, the macrolides may also be administered to the afflicted mammals by the topical or transdermal route to achieve either local or systemic effect. When used on animals, the compounds may be applied as a liquid drench. The animal drench is normally a solution, suspension or dispersion of the active compound, usually in water, together with a suspending agent such as bentonite and a wetting agent or similar excipient. Generally, the drenches also contain an antifoaming agent. Drench formulations typically contain about 0.001% to about 0.5%, by weight, of the active macrocyclic compound. Preferred drench formulations contain about 0.01% to about 0.1%, by weight.

Additionally, the macrocyclic compounds may be administered by applying as a gel, lotion, solution, cream or ointment to human skin or pouring on animal skin or hide via a solution. The topical or transdermal formulations comprise the active ingredient in combination with conventional inactive excipients and carriers. The cream, for example, may use liquid petrolatum, white petrolatum, propylene glycol, stearyl alcohol, cetyl alcohol, sodium lauryl sulfate, sodium phosphate buffer, polysorbates, parabens, emulsifying wax, polyoxyethylene-polyoxypropylene block copolymers, purified water and the like. Ointments, for example, may employ petrolatum, mineral oil, mineral wax, glycerin and the like. Topical solutions may provide the active ingredient compounded with propylene glycol, parabens, hydroxypropyl cellulose, preservatives. Pour-on formulations may constitute the active ingredient dissolved in a suitable inert solvent, such as dimethylsulfoxide, propylene glycol, butoxyethoxyethanol and the like. A particularly useful pour-on formulation comprises the active ingredient dissolved or dispersed in an aromatic solvent, PPG-2 myristyl ether propionate, polybutene, an antimicrobial agent, an antioxidant and a nontoxic pharmaceutically acceptable mineral or vegetable oil.

To increase the efficacy of the macrolides as pesticidal agents, the multidrug resistance reversing agents are applied to crops, crop seeds or the soil or water in which crops or seeds are growing or to be grown in a pesticidal enhancing effective amount. The MDR-reversing agents may be applied either before or concurrently with the application of the macrocyclic lactone. Typically, the MDR-reversing agent will be applied within 4 hours before or, preferably, concomitantly with the application of the macrocyclic lactone.

In terms of application rates, the suitable amount of the MDR-reversing agent which is effective to increase the efficacy of the macrocyclic lactone compound against resistant crop pests will typically vary within a wide range of amounts at a variety of concentrations and rates. The particular MDR-reversing agent selected for use with the crop pesticide will clearly affect the application rate of the MDR-reversing agent. It is contemplated that choice of appropriate amounts, concentrations, spray rates and the like of each MDR-reversing agent and the macrocyclic lactone compound to achieve the pesticidal enhancing effective amount can be easily determined by routine procedures known to those having ordinary skill in the agricultural art.

As insecticidal, nematocidal or acaricidal agents useful for protecting crop seeds or growing or harvested crops from the pest's attack, the compounds of the present invention may be formulated into dry compacted granules, flowable compositions, wettable powders, dusts, dust concentrates, microemulsions and the like, all of which lend themselves to soil, water or foliage application and provide the requisite plant protection. Such compositions include the compounds of the invention admixed with agronomically acceptable solid or liquid carriers.

In the agricultural composition, the active compounds are intimately mixed or ground together with the excipients and carriers in sufficient amounts to typically provide from about 3% to about 20% by weight of the macrocyclic lactone compound in said composition.

The compositions of this invention are useful in combatting agricultural pests that inflict damage upon crops while they are growing or while in storage. The compounds are applied using known techniques such as sprays, dusts, emulsions, wettable powders, flowables and the like to the growing or stored crops to provide protection against infestation by agricultural pests.

Unexpectedly, it is found that the mechanism of resistance to the macrocyclic lactone compounds is due to overexpression of novel P-glycoprotein homologs which causes an efflux of anthelmintic from the parasite. The present invention illustrates the involvement of the Pgp homolog genes in IVM resistance in *H. contortus*. The overexpression of Pgp-protein in IVM resistant strains of *H. contortus* is shown to be regulated by both rearrangement of genomic DNA encoding the PGP homologs and by gene transcription.

*H. contortus* in jirds (*Meriones unguiculatus*) has been used for the evaluation of anthelmintic efficacy and has been shown to correlate well with studies of this parasite in sheep (Conder et al., J. Parasitol. 78: 492-497, 1992). Employing the jird model, the present invention determines that multidrug reversing agents can unexpectedly be used to increase the efficacy of macrocyclic lactones against resistant parasites. As a representative example, the multidrug resistance (MDR) reversing agent verapamil (VRP) is shown to uniquely enhance the action of moxidectin and ivermectin against moxidectin susceptible and resistant *H*. *contortus*.

Parasites such as *H. contortus* contain Pgp homolog genes which are expressed in different stages of the parasite life cycle. This invention finds that the level of expression of P-glycoprotein is surprisingly elevated in different strains that are resistant to macrocyclic lactones such as ivermectin compared with the levels in the susceptible strains from which the resistant strains are derived. The higher level of Pgp expression, in ivermectin resistant strains, is associated with an alteration at the genomic level.

P-glycoproteins can act as molecular pumps to efflux hydrophobic xenobiotics from cells. An elevation in the level of the P-glycoproteins is the basis of multidrug resistance in cancer cells and also appears to be involved in some forms of drug resistance in some protozoa. An elevated level of Pgp has not so far been described as the mechanism of drug resistance in nematode parasites. This is the first evidence that shows that ivermectin resistance can be due to an elevation in P-glycoproteins. Ivermectin resistance is becoming a common problem in nematode parasites of animals and potentially in arthropod parasites. Its continued use against arthropods is likely to lead to the selection of similar resistance to that in nematodes.

Evidence exists that ivermectin shares a common action with other avermectins, such as doramectin, milbemycins (Arena *et al*., 1995) and moxidectin. It can be predicted that the development of resistance against other macrocyclic lactone compounds will involve hyperexpression of P-glycoprotein leading to elevated rates of drug efflux.

This work is significant because it allows the sensitive detection of ivermectin resistance and resistance to other macrocyclic lactone compounds in nematodes and arthropods using DNA and cDNA probes based on the demonstrated differences found in the *Pvu*II digests of DNA from resistant and susceptible organisms. It also permits the prediction of the degree of resistance from the level of P-glycoprotein expression based on either Pgp mRNA or Pgp protein levels. This understanding of the mechanism of resistance to the macrolides allows active analogs to be synthesized which will remain effective in the presence of an *mdr*-based mechanism of resistance to other macrocyclic lactones. More specifically, chemicals which act on the mode of action receptor, the glutamate-gated chloride channel (Arena *et al*., 1995), but which are not efficiently effluxed by the P-glycoprotein pump, i.e., are poor substrates for Pgp efflux, can be selected to overcome resistance. This will lead to improvements in parasite controls, especially in the prevention and treatment of ivermectin resistance and cross-resistance to other avermectins, milbemycins and the LL-F28249 compounds.

This invention provides new evidence that in resistance to macrocyclic lactone endectocides, such as ivermectin, in nematode and arthropod parasites of animals, expression of P-glycoprotein is elevated compared with the level of expression in the parental susceptible strains of the parasite. It further shows that the higher level of expression is associated with differences, at the genomic level, of P-glycoprotein genes. For example, using Southern blot analysis of pooled DNA and by PCR (Polymerase Chain Reaction) analysis of individual worms, differences are determined in the genomic DNA for Pgp in *Haemonchus contortus* resistant to ivermectin compared with the susceptible parental strain, and the allele diversity for Pgp in resistant worms appears to be markedly reduced compared with the parental susceptible strain. Novel nucleic acid probes which can differentiate between susceptible and resistant parasites are now found and deemed to be useful in the early detection of the development of resistance to macrocyclic lactone compounds.

For the first time, this invention demonstrates that macrolactone resistance can be overcome by using a MDR-reversing agent. For example, verapamil, a well-known, relatively weak MDR-reversing agent, significantly increases the efficacy of moxidectin against moxidectin resistant *H*. *contortus.* The moxidectin resistant worms show side resistance to ivermectin and the ivermectin resistance is also overcome with the use of a mild MDR-reversing agent.

The following examples demonstrate certain aspects of the present invention. However, it is to be understood that these examples are for illustration only and do not purport to be wholly definitive as to conditions and scope of this invention. It should be appreciated that when typical reaction conditions (e.g., temperature, reaction times, etc.) have been given, the conditions which are both above and below the specified ranges can also be used, though generally less conveniently. The examples are conducted at room temperature (about 23°C to about 28°C) and at atmospheric pressure. All parts and percents referred to herein are on a weight basis and all temperatures are expressed in degrees centigrade unless otherwise specified.

A further understanding of the invention may be obtained from the following non-limiting examples.

### EXAMPLE 1

### PCR Synthesis and Cloning of a 432 bp DNA for a P-Glycoprotein Homolog From a cDNA Library of H. contortus

Based on the highly conserved ATP binding domains of *C*. *elegans* Pgp, a pair of degenerate PCR primers is designed. The sense primer is 5'-ACNGTNGCNYTNGTNGG-3' (which corresponds to SEQ ID NO:11) and the antisense primer is 5'-GCNSWNGTNGCYTCRTC-3' (which corresponds to SEQ ID NO:12). PCR is carried out for 40 cycles at a denaturing temperature of 94°C for 1 minute, an annealing temperature of 37°C for 1 minute, and an extension temperature of 72°C for 3 minutes using an *H. contortus* cDNA library (Geary et al., Mol. Biochem. Parasitol., 50: 295-306, 1992) as template. A 432 bp product is purified by agarose gel electrophoresis and the purified product is used as template for a second round of PCR amplification with the same primers. An enriched 432 bp product is subsequently cloned into TA vector (Invitrogen) according to standard protocols. Plasmids with inserts are transformed into *Escherichia coli* and then plated on Ampicillin LB plates containing a chromogenic substrate, X-GAL® (5-bromo-4-chloro-3-indolyl-β-D-galactopyranoside, commercially available from Gibco BRL, Bethesda, MD) (Sambrook et al., Molecular Cloning. A Laboratory Manual, 2nd Ed., Cold Spring Harbor Laboratory Press, 1989). Ten clones are identified as ATP binding domain sequences of P-glycoprotein.

### EXAMPLE 2

### Screening of the H. contortus cDNA Library

The 432 bp fragment is excised by *EcoR*1*,* labelled by random priming with [³²P]d-CTP and used as a probe to screen the cDNA library (Sambrook *et al*., 1989). Approximately one million clones are screened and nine putative clones are identified. The positive clones are digested with *Pvu*II and three of them containing inserts in the predicted size are subsequently sequenced.

### EXAMPLE 3

### Parasite Strains

Two pairs of ivermectin susceptible and resistant strains of *H. contortus* are used. The first pair is an ivermectin resistant strain (MFR) developed at the Merck Research Laboratories, Rahway, NJ (Rohrer et al., J. Parasitol. 80: 493-497, 1994) and the ivermectin susceptible parent strain (MFS) from which the resistant strain is selected over seventeen generations of ivermectin selection. The second pair is an ivermectin resistant strain (ACR) developed at American Cyanamid Company, Princeton, NJ and the ivermectin susceptible parent strain (ACS) from which the resistant strain is selected over fourteen generations of ivermectin selection. Strain MFR is reported to be 10X resistant at the ED₉₅ compared with MFS, and ACR, after twelve generations of selection, is found to be 6.3X resistant at the ED₉₅ compared with ACS.

### EXAMPLE 4

### RNA Extraction and Northern Hybridization

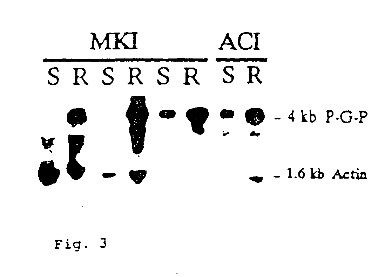

Adult worms from ivermectin susceptible and resistant *H*. *contortus* are collected from the abomasum of sheep (Lubega and Prichard, Biochem. Pharmacol. 41: 93-101, 1991). Eggs from each strain are collected and isolated from faeces of sheep (Weston et al., J. Parasitol. 14: 159-164, 1984) which have been previously worm free and inoculated with one of the four *H. contortus* strains. Total RNA is extracted from tissues of the ivermectin susceptible and resistant strains, respectively, using TRIzoL® Reagent (Gibco BRL Life Technologies, Inc., Gaithersburg, MD, company protocol). Total RNA is run on denaturing formaldehyde agarose gel electrophoresis and transferred to H-bond nylon membranes. The membranes are prehybridized at 65°C in 10% dextran disulfate, 1% SDS (sodium dodecylsulfate), 1.0M NaCl over 4 hours. The ³²P-labelled 432 bp *H, contortus* Pgp fragment and an actin probe consisting of the 1.25 kb *Pst*I fragment from *pBA*1 (Degen et al., J. Biol. Chem. 258: 12153, 1983) are mixed and incubated overnight with the membranes at 65°C in the same hybridization buffer. The membranes are washed with 2X SSC (1:2 mixture of trisodium citrate and sodium chloride), 0.1% SDS at 65°C for 30 minutes and 0.5X SSC at 35°C for 1 hour and then autoradiographed. Image analyses of gel autoradiographs are made for quantitative determination of mRNA expression, using the IMAGE program (O'Neil et al., Appl. Theor. Electrophor., 1: 163-167, 1989). Actin DNA probes from a mouse source, labelled and hybridized with the same blot, using the same method as above, is used as an internal control for mRNA loading. Results are shown in Fig. 3 (S = unselected strains; R = IVM selected strains; MKI = strains developed at Merck Research Laboratories, Rahway, NJ; ACI = strains developed at American Cyanamid Company, Princeton, NJ).

### EXAMPLE 5

### Southern Blots

Genomic DNA from both ivermectin susceptible and resistant strains is extracted (Sambrook *et al.,* 1989). Four restriction enzymes *EcoR*I, *Cla*I*, Pvu*II and *Pst*I are used to digest the genomic DNA following the suppliers' directions. Each reaction is carried out with both ivermectin resistant and susceptible strains. After an overnight restriction enzyme digestion, samples are run on 1% agarose gels and then blotted onto H-bond membranes (Sambrook *et al*., 1989). The membranes with DNA are exposed under UV light to fix the DNA to the membranes. The membranes are prehybridized at 65°C in buffer (10% sulfur dextran, 1% SDS and 1M NaCl) for at least 4 hours. The 432 bp fragment, labelled with [³²P], is added as a probe and hybridized with the genomic DNA in the prehybridization buffer, overnight. The membranes are subsequently washed twice with 2X SCC for 10 minutes, twice with 1X SCC for 15 minutes and then autoradiographed.

### RESULTS

### PCR Amplification

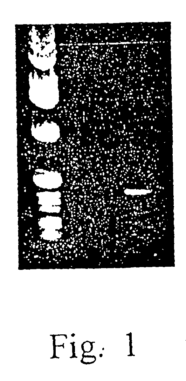

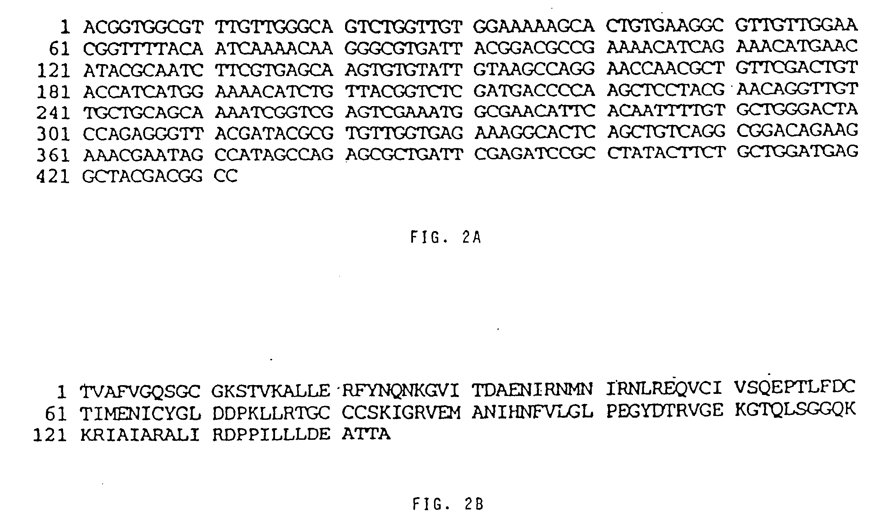

Two rounds of PCR amplification generate a 432 bp product (Fig. 1) which is highly homologous to the conserved ATP binding domain of P-glycoprotein (Fig. 2A). The putative amino acid sequence (Fig. 2B) shows that this fragment is highly homologous to P-glycoprotein or multiple drug resistant proteins from *C. elegans,* mouse and other species. These data indicate that the 432 bp fragment represents the ATP binding sequence of an *H. contortus* Pgp homolog.

### Expression of P-Glycoprotein mRNA in Ivermectin Resistant and Susceptible Strains of H.contortus

A single animal species may have different Pgp which may vary in size. Northern hybridization, with the 432 bp Pgp *H*. *contortus* homolog PCR product, shows that the molecular size of the mRNA for the *H. contortus* Pgp is about 4 kb. However, it is found that the mRNA levels of Pgp in ivermectin resistant and susceptible strains of *H*. *contortus* are different. For illustration, results of a number of representative Northern blots on *H. contortus* egg RNA are shown in Fig. 3.

The RNA is also probed with an actin probe to allow correction for different amounts of RNA loaded onto the gels. The intensity of the Pgp mRNA band varies with the strain of parasite. After correction for the intensity of the actin band, it is found that the amount of the 4 kb mRNA band recognized by the 432 bp Pgp probe is much higher in both ivermectin resistant strains compared with their respective ivermectin susceptible precursor strains. The increase varies from 250% to 670% after standardization for actin mRNA expression in drug resistant and susceptible strains (Table 1). Similar results are also obtained in comparisons of Pgp expression using RNA extracted from adult *H. contortus.*

Table 1 shows the relative intensity of mRNA for P-glycoprotein and actin in ivermectin susceptible and resistant *Haemonchus contortus* strains. RNA is extracted from eggs from the respective Merck (MKI) and American Cyanamid (ACI) paired strains. Each susceptible and resistant pair are processed at the same time. The RNA is separated on an agarose gel and probed with both *H. contortus* 432 bp Pgp and the actin *pBA*1 radiolabelled probes. The relative intensity of each band is determined, after gel autoradiography, by gel densitometry. The intensity of each Pgp band is corrected for intensity of its corresponding actin band in order to adjust for different amounts of RNA having been loaded onto the gels. All comparisons are made by pairs (resistant (R) *versus* corresponding susceptible (S)).

**TABLE 1**

| Strains comparison | Corrected R/S ratio |
|---|---|
| MKIS/MKIR | 6.77 |
| MKIS/MKIR | 6.08 |
| MKIS/MKIR | 2.57 |
| ACIS/ACIR | 4.19 |

### Sequencing of P-Glycoprotein Homologs From the H. contortus cDNA Library

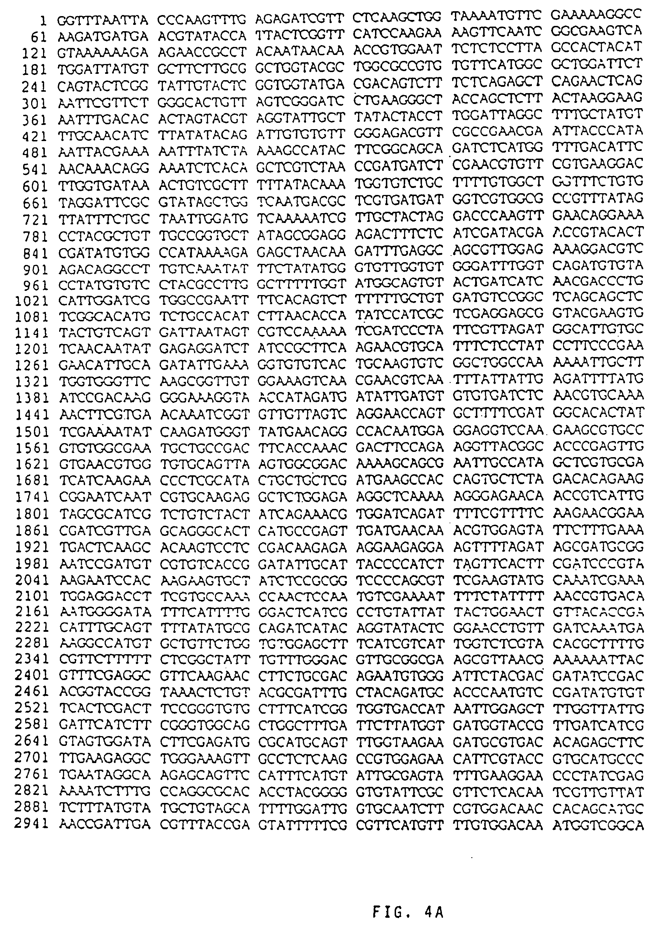

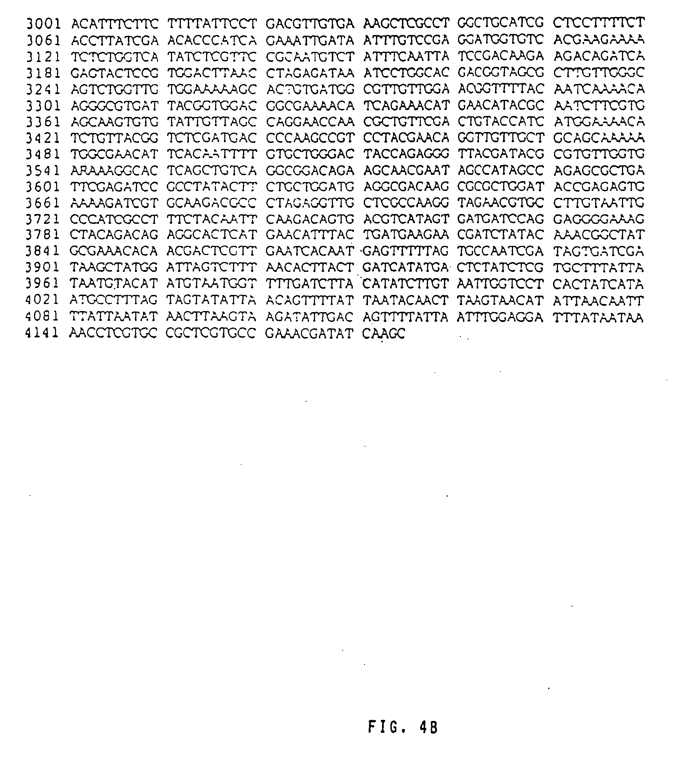

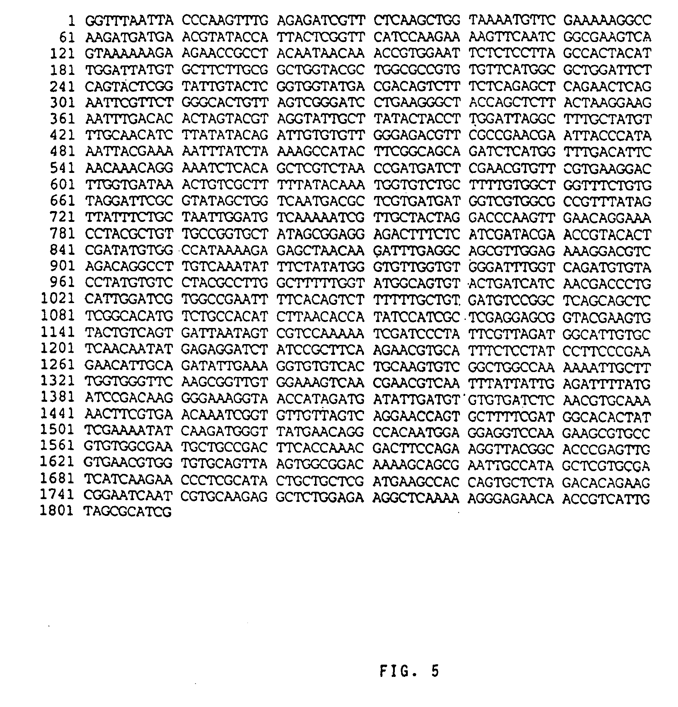

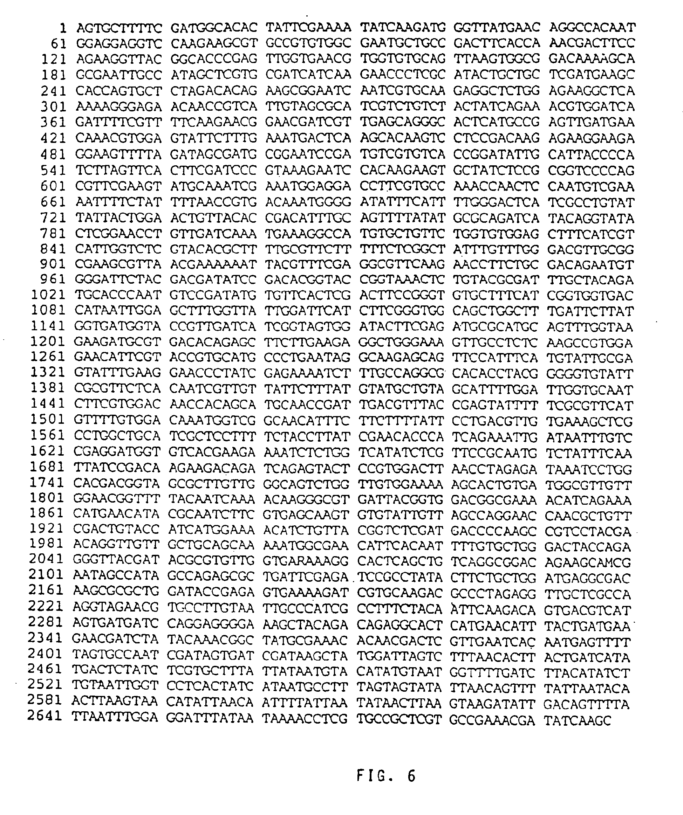

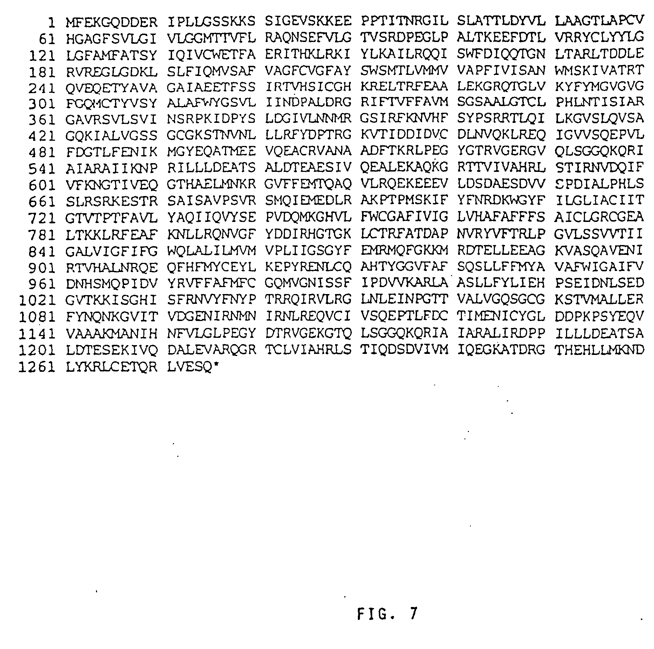

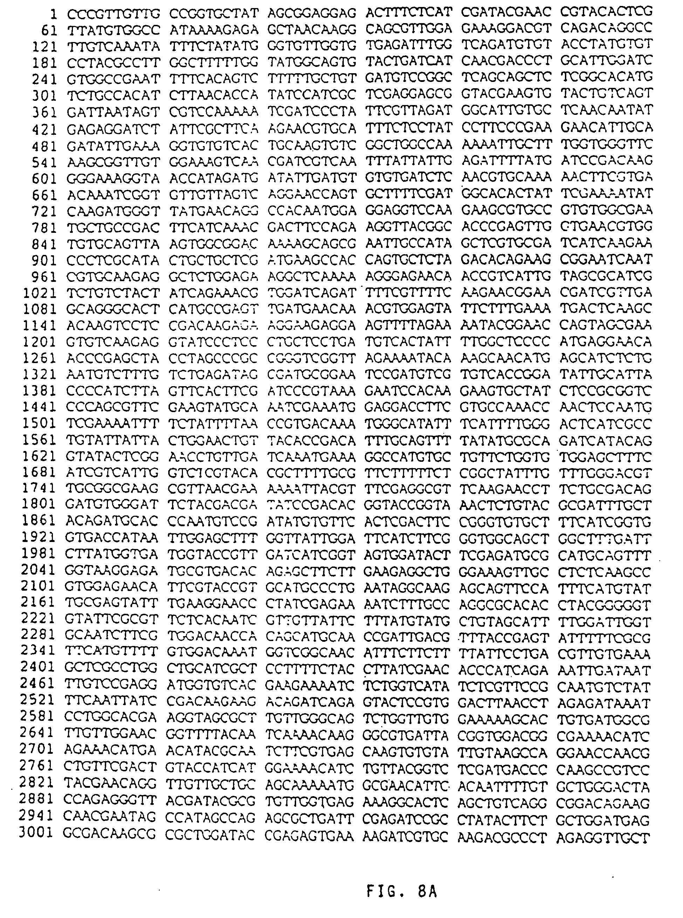

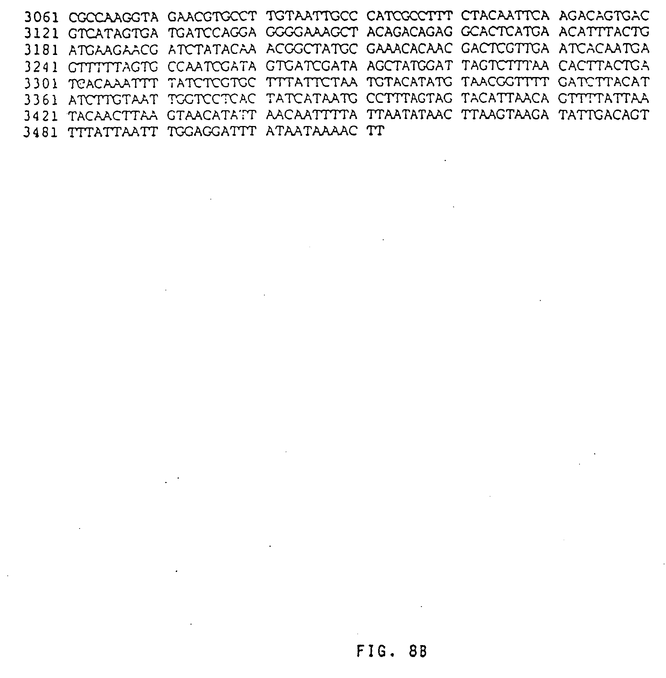

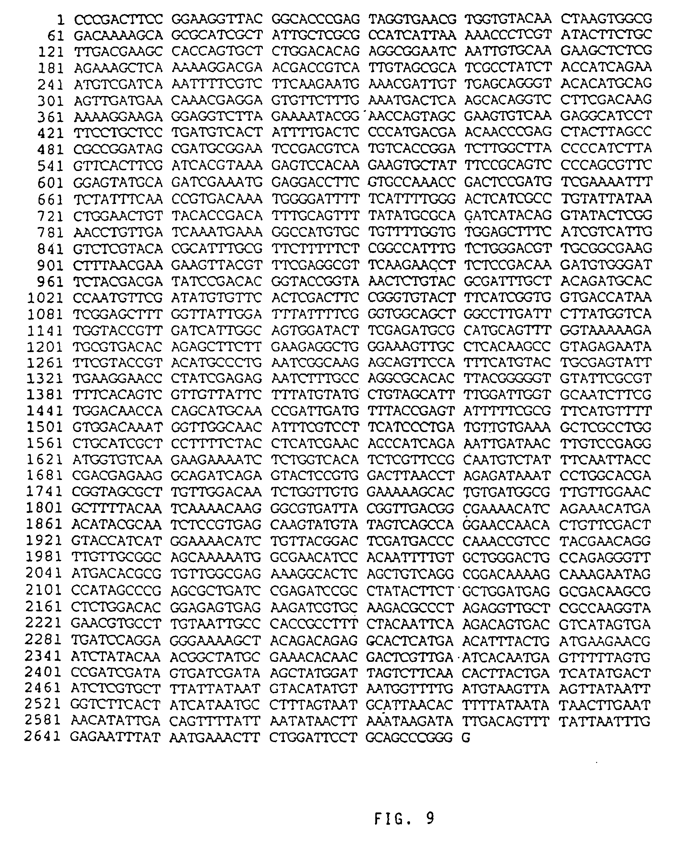

Longer clones (4.2 kb, 3.5 kb and 2.7 kb) identified using the 432 bp probe, which are shown to be homologous to P-glycoprotein, are fully or partially sequenced. Figures 4A to 4B show the full cDNA sequence for the PGP-A clone (4175 bp) which has high homology to known P-glycoprotein genes such as the *Xenopus* putative multidrug resistance protein *(Xemdr)* and the *C. elegans cepgp*A gene for P-glycoprotein A. Figures 5 and 6 show the partial sequence, in the sense direction (Fig. 5; PGP-A-5') and the antisense direction (Fig. 6; PGP-A-3') of the cDNA fragment which is also highly homologous to P-glycoprotein. Figures 7-9 illustrate, respectively, the putative amino acid translation of PGP-A cDNA, the partial cDNA sequence of the 3' end of the PGP-O clone (3.5 kb), antisense direction, and the partial cDNA sequence of 3' end of the PGP-B clone (2.7 kb), antisense direction.

### Genomic DNA Differences Between Ivermectin Resistant and Susceptible Strains of H. contortus and Determination of a Nucleic Acid Probe for the Detection of Macrolactone Susceptibility or Resistance

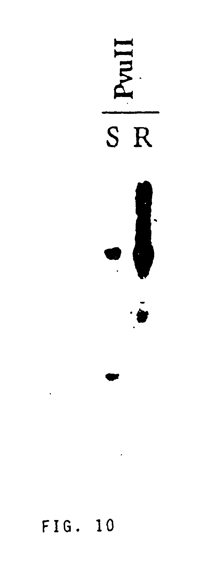

Genomic DNA hybridizations show that at least two bands are recognized by the 432 bp probe in *Cla*I and *Pst*I digestion maps of both ivermectin susceptible and resistant strains. The *EcoRI* digestion maps show three strongly hybridizing bands and one light band for both susceptible and resistant strains. However, the *Pvu*II digestion patterns are clearly different between the ivermectin resistant and susceptible strains (Fig. 10).

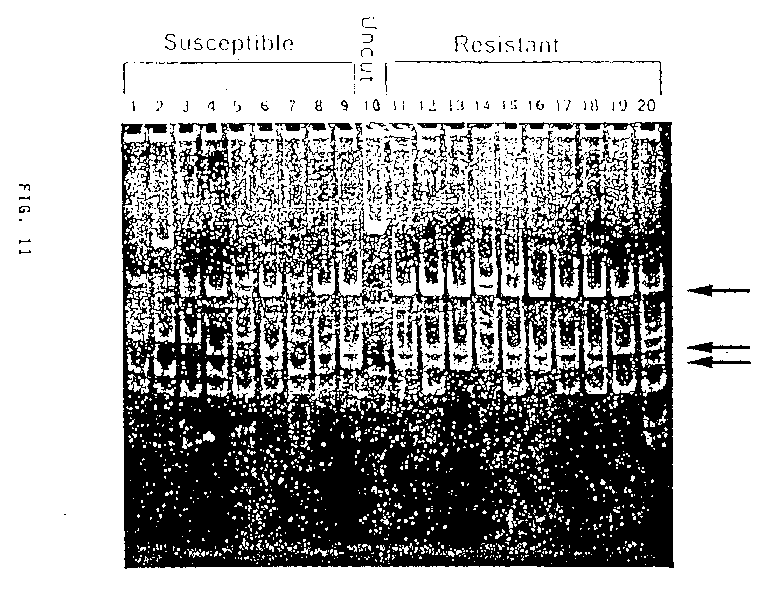

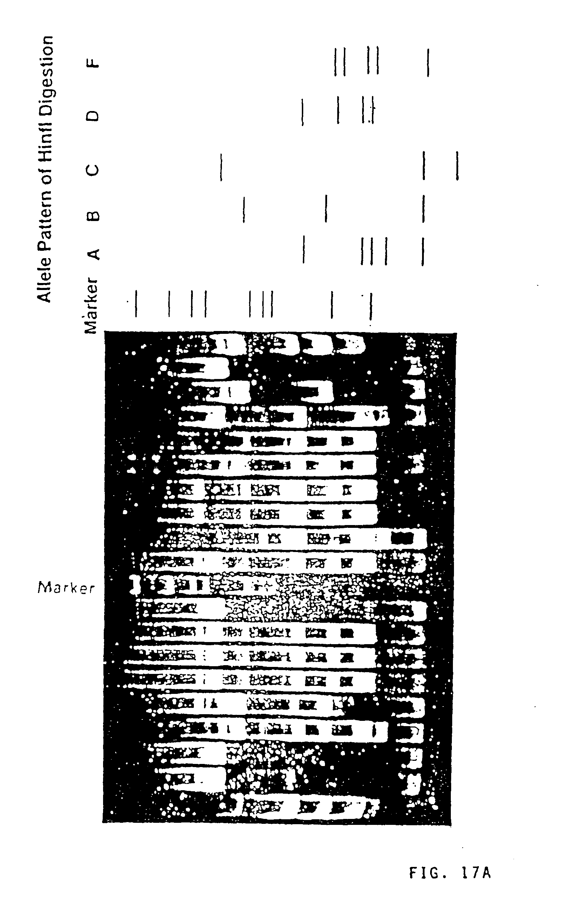

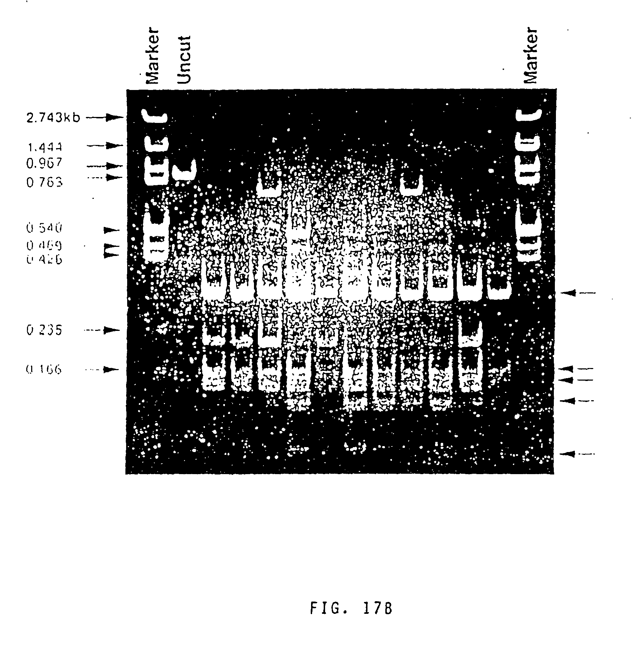

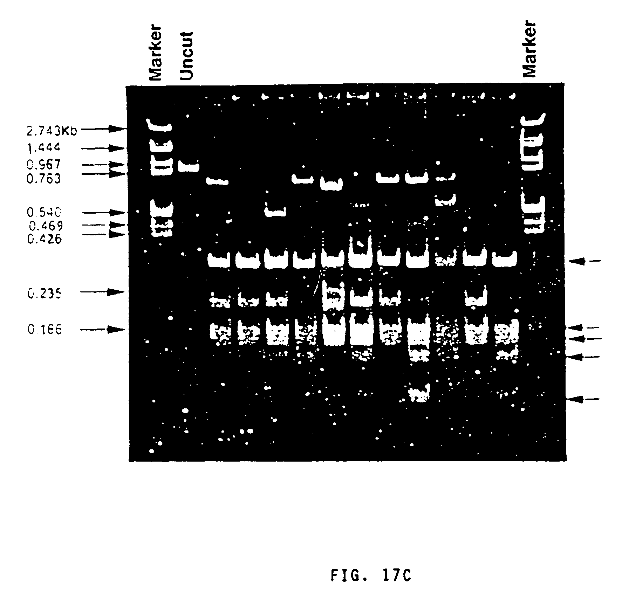

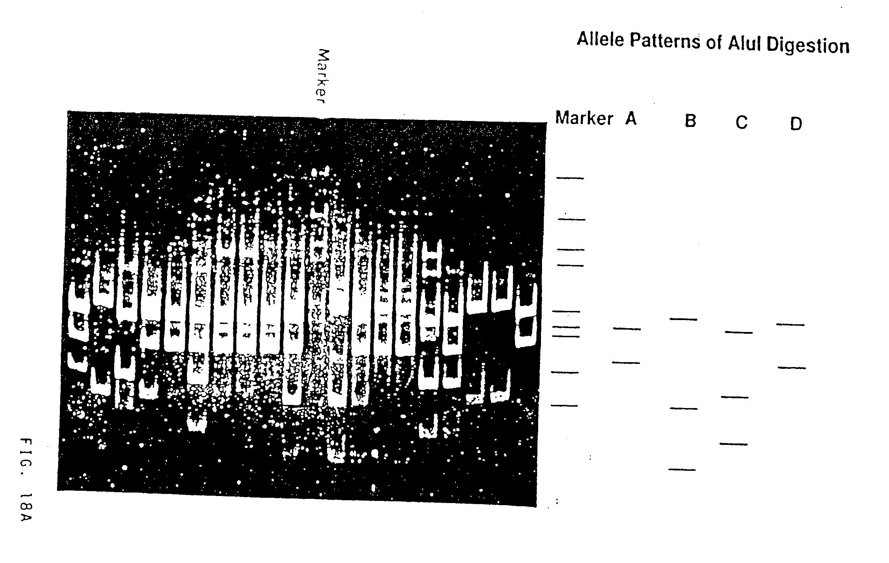

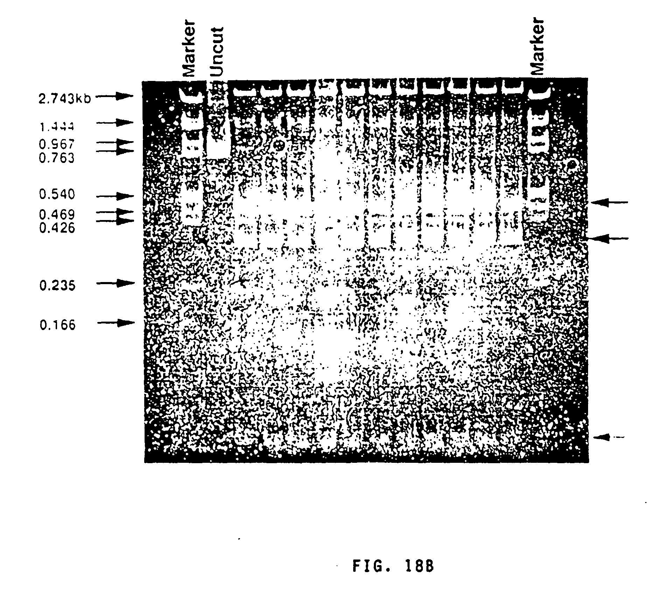

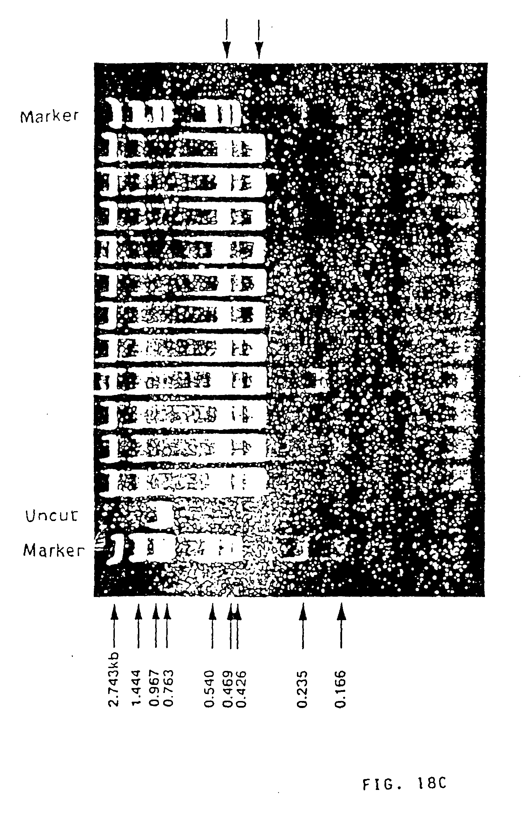

PCR products are generated using pairs of primers which are specific to parasite Pgp genes. In one example, the reverse primer is specific for a region 53 base pairs in length present in one of the Pgp clones (PGP-O). The forward primer anneals to a region common to multiple Pgp clones. Genomic DNA extracted from individual male *H. contortus* adults from IVM-sensitive (24 worms) and IVM-resistant (29 worms) populations (MKIS and MKIR) is used as template for amplification by PCR. The Pgp PCR products, approximately 900 bp in length, are digested with the restriction enzyme *Dde*I and the digestion products are separated by non-denaturing polyacrylamide gel electrophoresis (Fig. 11; see also Figs. 17A-18C illustrating diagnostic restriction patterns for resistance after selection with either ivermectin or moxidectin, using different worm strains and different restriction enzymes). The digestion pattern for the worms from the susceptible population is variable, while that for the worms from the resistant population is more homogeneous. An identical digestion pattern of three bands (arrows) is found in 28 of the 29 worms from the resistant population (Fig. 11, lanes 11-18 and 20, for example), whereas only 4 or 5 worms from the susceptible population have this pattern (Fig. 11, lanes 6 and 9, for example). Examples of the probes are shown in Figures 12A and 12B.

These results are repeated several times. The PCR data and the Southern blot data clearly indicate that selection for macrocyclic lactone endectocide resistance causes a reduction in the genetic diversity of the Pgp alleles and that the differences in Pgp at the DNA level can be detected by specific probes techniques such as PCR (Polymerase Chain Reaction), Southern blot analysis and RFLP (Restriction Fragment Length Polymorphism).

### Additional Methods

### EXAMPLE 6

### Establishing the LD₅₀ for Moxidectin and Ivermectin Against Moxidectin Susceptible and Resistant H. contortus in the Jird

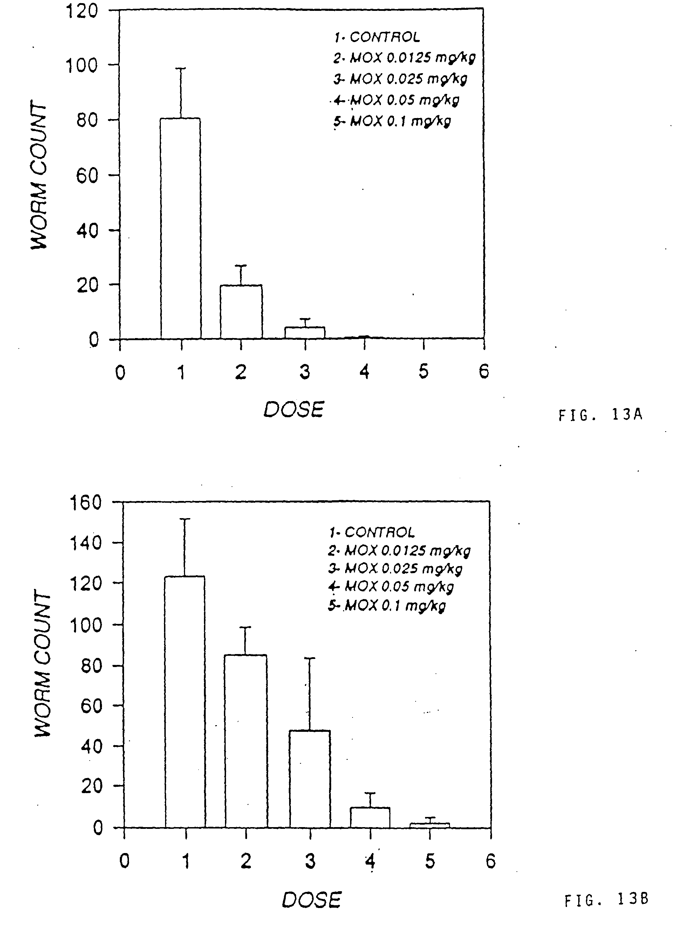

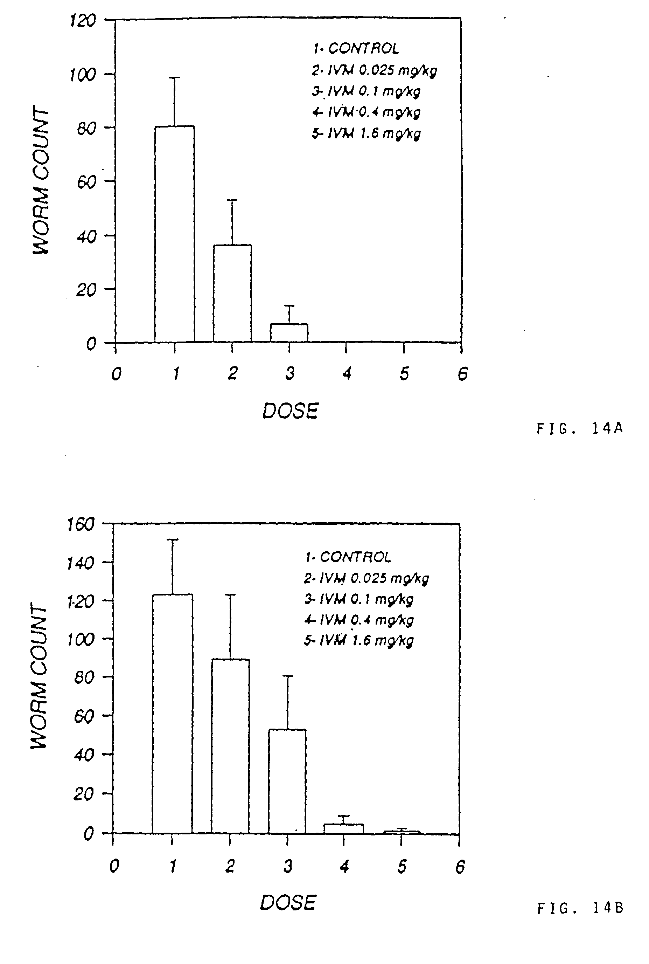

Jirds, which are fed on a standard commercial ration to which 0.02% hydrocortisone has been added 5 days prior to infection, are inoculated with 1000 exsheathed L₃ *H*. *contortus.* On day 10 after inoculation, the jirds are treated with either water or various doses of moxidectin or ivermectin orally. Each treatment group contains 6 jirds. The parasite strains and anthelmintic dose rates are shown in Table 2. The results of these dose titrations are shown in Figures 13A-14B. Probit analyses are used to estimate LD₅₀ levels for each anthelmintic against each strain. The estimated LD₅₀ of moxidectin against the susceptible and moxidectin resistant strains are 0.010 and 0.017 mg/kg, respectively, and for ivermectin the estimate LD₅₀ levels are 0.024 and 0.046 mg/kg, respectively.

The results indicate that (i) moxidectin is more potent than ivermectin against both the susceptible and moxidectin resistant strains, and (ii) moxidectin resistant *H. contortus* are side-resistant to ivermectin.

**Table 2. DOSE RATES (mg/kg) OF MOXIDECTIN (MOX) OR IVERMECTIN (IVM) AGAINST H. contortus MOX-RESISTANT AND SUSCEPTIBLE STRAINS IN JIRDS (n=6)**

| COMPOUND (mg/kg) | SUSCEPTIBLE (PF14) | RESISTANT (MOF14) |
|---|---|---|
| CONTROL | --- | --- |
| MOX | 0.0125 | 0.0125 |
| MOX | 0.025 | 0.025 |
| MOX | 0.05 | 0.05 |
| MOX | 0.1 | 0.1 |
| IVM | 0.025 | 0.025 |
| IVM | 0.1 | 0.1 |
| IVM | 0.4 | 0.4 |
| IVM | 1.6 | 1.6 |

### EXAMPLE 7

### Determination of the Toxicity and the Efficacy of Verapamil Alone and in Combination With Ivermectin

This experiment is performed to determine the toxicity of verapamil, a weak MDR-reversing agent, alone and in combination with ivermectin, the efficacy of verapamil alone against *H. contortus* and the effect of verapamil at 20 mg/kg on the efficacy of ivermectin against susceptible and moxidectin resistant worms. Dose rates of verapamil between 20 and 80 mg/kg are used alone or in combination with ivermectin at 0.024 and 0.046 mg/kg in jirds infected with susceptible or moxidectin resistant *H. contortus.* Verapamil is given concomitantly with ivermectin by the oral route. The results are shown in Table 3.

**Table 3. DEMONSTRATION OF TOXICITY AND EFFICACY OF VERAPAMIL (VRP) WITH OR WITHOUT IVERMECTIN AGAINST H. contortus MOXIDECTIN RESISTANT OR SUSCEPTIBLE STRAINS IN JIRDS (n = # per group)**

| COMPOUND (mg/kg) | # SUSCEPTIBLE (PF14) | # RESISTANT (MOF14) |
|---|---|---|
| CONTROL | 5 | 5 |
| VRP 20 | 5 | 5 |
| VRP 40 | 3 | 3 |
| VRP 60 | 3 | 3 |
| VRP 80 | 3 | 3 |
| IVM** | 5 | 5 |
| IVM/VRP 20 | 5 | 5 |
| IVM/VRP 40 | 5 | 5 |
| IVM/VRP 60 | 5 | 5 |
| IVM/VRP 80 | 5 | 5 |

| | | |
|---|---|---|
| ** The dose of ivermectin is 0.024 mg/kg against strain PF14 and 0.046 mg/kg against strain MOF14. | | |

As no deaths or other signs of toxicity are observed at a verapamil dose rate of 20 mg/kg, in the absence or presence of ivermectin, this dose rate is used for subsequent resistance reversing experiments. Verapamil alone is found to have no significant effect on worm counts at any of the dose rates used.

The toxicity of verapamil is summarized in Table 4.

**Table 4. TOXICITY OF VERAPAMIL TO JIRDS**

| VRP (mg/kg) | DEATHS - VRP ALONE | DEATHS - VRP + IVM* |
|---|---|---|
| 20 | 0/10 | 0/10 |
| 40 | 0/5 | 2/10 |
| 60 | 1/6 | 1/10 |
| 80 | 3/7 | 2/10 |

| | | |
|---|---|---|
| * Ivermectin is used at 0.24 mg/kg or 0.046 mg/kg according to Table 3. | | |

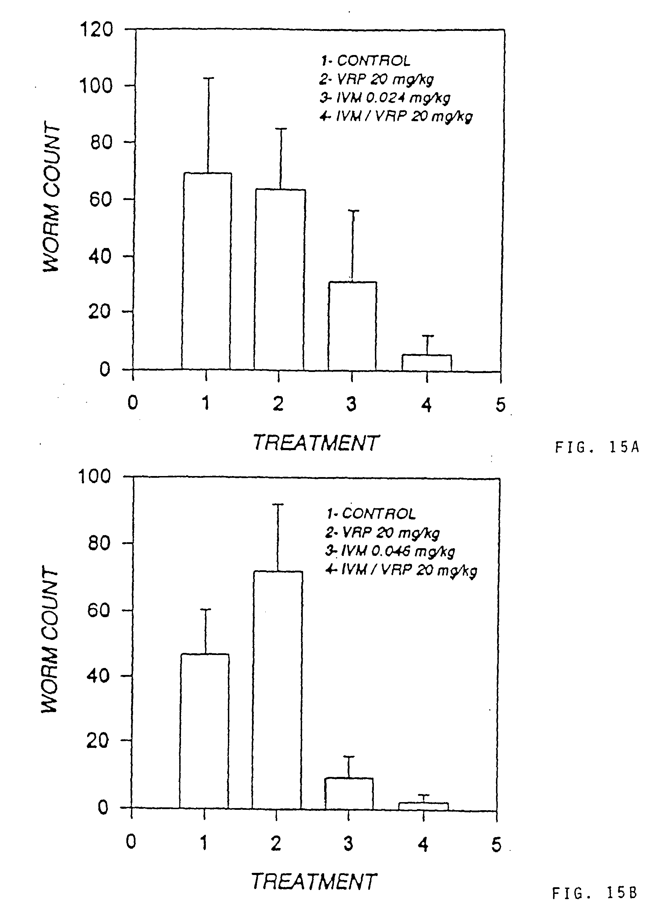

Because of the toxicity of verapamil at dose rates of 40 mg/kg and above, only the effects of verapamil at 20 mg/kg on the efficacy of ivermectin against susceptible and moxidectin resistant worms are considered. These results, shown in Figures 15A and 15B, are summarized in Table 5. Verapamil at 20 mg/kg significantly enhances the efficacy of ivermectin against the moxidectin resistant worms.

**Table 5. EFFECT OF VERAPAMIL (20 mg/kg) ON THE EFFICACY (%) OF IVERMECTIN**

| STRAIN | SUSCEPTIBLE (PF14) | MOX-RESISTANT (MOF14) |
|---|---|---|
| TREATMENT (mg/kg) | % EFFICACY* | % EFFICACY* |
| CONTROL | 0 | 0 |
| VRP 20 | 17 (n.s.) | -53 (n.s.) |
| IVM^{#} | 54 (A) | 79 (A) |
| IVM^{#}/VRP 20 | 92 (A) | 96 (B) |

| | | |
|---|---|---|
| # Ivermectin is administered at 0.024 mg/Kg to jirds infected with PF14 strain and at 0.046 mg/kg to jirds infected with the MOF14 strain. "n.s." indicates that the worm counts are not significantly different from the controls; "A" means significantly different from the controls, but not from other worm counts, of the same strain, with the same letter; "B" means significantly different worm counts from "A" for the same strain and dose rate of ivermectin. | | |

### EXAMPLE 8

### The Effects of Verapamil on the Efficacy of Moxidectin and Ivermectin Against Susceptible and Moxidectin Resistant H. contortus

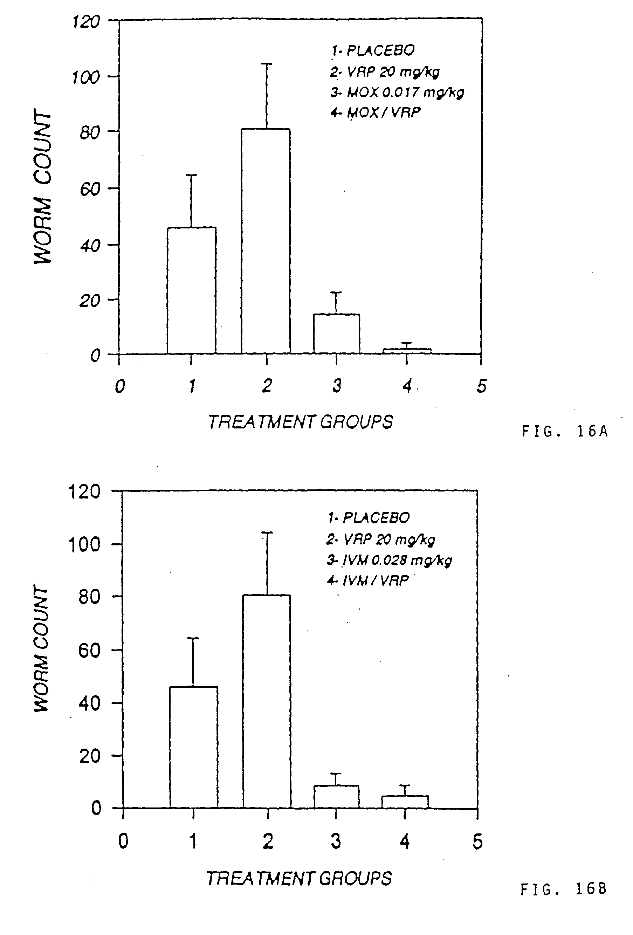

This experiment is performed on jirds to determine the effects of verapamil at 20 mg/kg on the efficacy of moxidectin and ivermectin against susceptible and moxidectin resistant *H*. *contortus.* All treatments have 7 jirds/group. The dose rates of moxidectin and ivermectin are selected to give approximately 50% efficacy in the absence of verapamil. Verapamil at 20 mg/kg significantly increases the efficacy of moxidectin against the resistant worms. The increase observed when verapamil is coadministered with ivermectin is not significant in this experiment as the efficacy obtained with ivermectin alone is already relatively high. The results are shown in Table 6 (see graphic representation of results in Figures 16A and 16B).

**Table 6. EFFECT OF VERAPAMIL ON THE EFFICACY (%) OF MOXIDECTIN AND IVERMECTIN AGAINST THE MOXIDECTIN-RESISTANT STRAIN OF H. contortus (MOF14)**

| TREATMENT | WORM COUNTS (MEAN ± S.E.) | EFFICACY (%) SIGNIFICANCE AT *P*<0.05 |
|---|---|---|
| PLACEBO | 46 ± 7 | --- A# |
| VRP* | 80 ± 9 | -73 A |
| MOX (0.017 mg/kg) | 14 ± 3 | 70 B |
| MOX (0.017 mg/kg) + VRP* | 2 ± 1 | 96 C |
| IVM (0.028 mg/kg) | 9 ± 1 | 80 B |
| IVM (0.028 mg/kg) + VRP* | 3 ± 1 | 93 B |

| | | |
|---|---|---|
| * Verapamil is administered at 20 mg/kg. All treatments are by the oral route. # Different letters indicate the mean worm counts are statistically different. However, the ivermectin (± verapamil) results are not compared with the moxidectin (± verapamil) results. Verapamil by itself increases worm counts, but the mean count is not statistically different from the control. | | |

This experiment confirms that the weak MDR-reversing agent verapamil overcomes resistance in nematodes to the macrolactones. These results are fully consistent with the above molecular evidence that macrolactone resistance is associated with the overexpression of P-glycoprotein homolog due to a change in P-glycoprotein DNA in resistant parasites. More potent MDR-reversing agents, such as cyclosporin A, SDZ-PSC 833 or other potent reversing agents can, at low dose rates, markedly increase the efficacy of macrocyclic lactone endectocides against resistant parasites.

In the foregoing, there has been provided a detailed description of particular embodiments of the present invention for the purpose of illustration and not limitation. It is to be understood that all other modifications, ramifications and equivalents obvious to those having skill in the art based on this disclosure are intended to be included within the scope of the invention as claimed.

## Claims

1. A method for increasing the efficacy of a macrocyclic lactone compound against a resistant crop pest **characterized by** applying to the crop, to the crop seed or to the soil or water in which the crop or the seed is growing or is to be grown a pesticidal enhancing effective amount of a multidrug resistance reversing agent.

2. A method for increasing the efficacy of a macrocyclic lactone compound against a resistant nematode or a resistant arthropod ectoparasite or endoparasite of a mammal **characterized by** administering to the mammal a pesticidal enhancing effective amount of a multidrug resistance reversing agent in connection with the administration of the macrocyclic lactone compound.

3. An improved composition for controlling or combatting a crop pest wherein the improvement comprises a pesticidally effective amount of a multidrug resistance reversing agent in combination with a macrocyclic lactone compound and an agronomically acceptable carrier.

4. An improved composition for controlling or treating helminth or arthropod endo- or ectoparasitic insect infestation or infection of a mammal wherein the improvement comprises an anthelmintically or an arthropod endo- or ectoparasiticidally effective amount of a multidrug resistance reversing agent in combination with a macrocyclic lactone compound and a nontoxic pharmaceutically acceptable carrier.

5. An improved method for controlling or combatting a crop pest wherein the improvement comprises applying to the crop, to the crop seed or to the soil or water in which the crop or the seed is growing or is to be grown a pesticidally effective amount of the composition of Claim 3.

6. An improved method for controlling or treating helminth or arthropod endo- or ectoparasitic insect infection or infestation of a mammal wherein the improvement comprises administering to the mammal to be treated an anthelmintically or an arthropod endo- or ectoparasiticidally effective amount of the composition of Claim 4.

7. A purified and isolated nucleic acid molecule or a fragment thereof, **characterized in that** said nucleic acid molecule or said fragment is extracted from a nematode or an arthropod pest and encodes a P-glycoprotein homolog which regulates resistance to a macrocyclic lactone compound.

8. A biologically functional plasmid or viral vector **characterized by** containing the nucleic acid molecule or the fragment according to Claim 7.

9. A suitable host cell stably transformed or transfected by a vector **characterized by** comprising the nucleic acid molecule or the fragment according to Claim 7.

10. A process for the production of a polypeptide product having part or all of the primary structural conformation and the biological activity of a P-glycoprotein homolog product, said process **characterized by** comprising: growing, under suitable nutrient conditions, procaryotic or eucaryotic host cells transformed or transfected with the nucleic acid molecule or the fragment according to Claim 7 in a manner allowing expression of said polypeptide product, and isolating the desired polypeptide product of the expression of said nucleic acid molecule or said fragment.

11. A P-glycoprotein homolog product of the expression in a procaryotic or eucaryotic host cell **characterized in that** the protein product is encoded by the nucleic acid molecule or the fragment according to Claim 7.

12. A recombinant nucleic acid molecule or a fragment thereof **characterized in that** said recombinant nucleic acid or the fragment encodes a P-glycoprotein homolog which regulates resistance to a macrocyclic lactone compound.

13. The nucleic acid molecule or the fragment according to Claim 7 or 12, **characterized by** having a nucleotide sequence encoding PGP-A set forth in SEQ ID NO:3, PGP-A-3' set forth in SEQ ID NO:5 (ATCC accession number 98336), PGP-B, PGP-B-3' set forth in SEQ ID NO:8 (ATCC accession number 98307), PGP-O or PGP-O-3' set forth in SEQ ID NO:7 (ATCC accession number 98309); the complementary strands thereof or a nucleotide sequence which hybridizes at about 65°C in the presence of a dextran buffer over at least about 4 hours to the nucleotide sequence encoding PGP-A, PGP-A-3', PGP-B, PGP-B-3', PGP-O or PGP-O-3'.

14. A method for detecting the resistance to a macrocyclic lactone compound in a nematode or an arthropod pest **characterized by** comprising: comparing a nucleic acid molecule extracted from a pest specimen to a nucleic acid molecule encoding for resistance to the macrocyclic lactone compound and a nucleic acid molecule encoding for susceptibility to the macrocyclic lactone compound.

15. The method according to Claim 14, **characterized by** hybridizing the nucleic acid molecule extracted from the pest specimen with a nucleic acid probe having a nucleotide sequence encoding PGP-A set forth in SEQ ID NO:3, PGP-A-3' set forth in SEQ ID NO:5 (ATCC accession number 98336), PGP-B, PGP-B-3' set forth in SEQ ID NO:8 (ATCC accession number 98307), PGP-O or PGP-O-3' set forth in SEQ ID NO:7 (ATCC accession number 98309); the complementary strands thereof or a nucleotide sequence which hybridizes at about 65°C in the presence of a dextran buffer over at least about 4 hours to the nucleotide sequence encoding PGP-A, PGP-A-3', PGP-B, PGP-B-3', PGP-O or PGP-O-3'.

16. The method according to Claim 14, **characterized by** mixing one to three of the nucleic acid molecules with a Polymerase Chain Reaction (PCR) or a Reverse Transcriptase Polymerase Chain Reaction (RT-PCR) primer; and the PCR or RT PCR primer comprises a nucleotide sequence between PGP2S and PGPAS in the sense and antisense directions, respectively, set forth in Figures 12A and 12B or a nucleotide sequence encoding 2PGP-A set forth in SEQ IDNO:3, PGP-A-3' set forth in SEQ ID NO:5 (ATCC accession number 98336), PGP-B, PGP-B-3' set forth in SEQ ID NO:8 (ATCC accession number 98307), PGP-O or PGP-O-3' set forth in SEQ ID NO:7 (ATCC accession number 98309); the complementary strands thereof or a nucleotide sequence which hybridizes at about 65°C in the presence of a dextran buffer over at least about 4 hours to the nucleotide sequence encoding PGP-A, PGP-A-3', PGP-B, PGP-B-3', PGP-O or PGP-O-3'.

17. A method for detecting the resistance to a macrocyclic lactone compound in a nematode or an arthropod pest **characterized by** comprising: preparing an antibody to a sequence of a peptide corresponding to the amino acid translation of a nucleic acid molecule or a fragment thereof encoding a P-glycoprotein homolog which regulates resistance to a macrocyclic lactone compound; preparing a specimen of the nematode or the arthropod pest, or an extract thereof, for reaction with the antibody; reacting the specimen or the extract with the antibody under suitable conditions that allow antibody-antigen binding to occur; and testing for the presence of the antibody-antigen binding.
